# EUROPEAN PATENT APPLICATION

(11) **EP 3 098 307 A1**
(43) Date of publication of application: **30.11.2016**
(21) Application number: 15181335.9
(22) Date of filing: 18.08.2015
(51) Int. Cl.: C12N 5/0789, A61K 35/28

(54) **A CULTURE MEDIUM FOR HEMATOPOIETIC STEM CELLS AND THE APPLICATIONS THEREOF AS WELL AS THE STEM CELLS CULTIRE METHOD**

(30) Priority: 28.05.2015 CN 201510283776
(71) Applicant: Shenzhen Fulixin Health Industry Development Limited, Shenzhen, Hong Kong 51800 (CN); China Life Science Technology Group Limited, Wanchai, Hong Kong (CN)
(72) Inventor: LIU, Xiaoqing, 200072 Shanghai (CN)
(74) Representative: Heinze, Ekkehard

(57) **Abstract**

The present invention discloses a culture medium for hematopoietic stem cells and the applications thereof as well as the stem cells culture method, which adopts amino acids, vitamins, salts, lipids, protein polypeptides and cytokines, together with one or several of trace elements, small organic molecules, hematopoietic stem cell proliferation agents, plant extracts and animal tissue cell extracts, to compose the hematopoietic stem cell culture medium, which can quickly proliferate different species of CD34+ cells, and improve both the proliferation speed and telomere length of CD34+ cells significantly; also, hematopoietic stem cells cultured in the hematopoietic stem cell culture medium described in the present invention may keep their cell functions as well, achieve the functions include but not limited to anti-tumor, anti-aging, and extending lives, thus having a pretty good application prospect.

## Description

The present invention relates to the field of cell culture technologies, and more particularly, to a culture medium for hematopoietic stem cells and the applications thereof as well as the stem cells culture method.

Numerous studies have shown that hematopoietic stem cells play a key role in the body fluid circulations: on one hand, red blood cells, lymphocytes in the body fluid are both derived from hematopoietic stem cells, however, under normal physiological conditions, hematopoietic stem cells are stored primarily in bone marrows and only in emergency conditions or being processed by mobilization agents will they be released into the peripheral blood flow; on the other hand, the cells renewal in body fluids also relies on the amount of hematopoietic stem cells and their differentiation potentials. With the rise of personalized regenerative medicine technologies, hematopoietic stem cells play an increasingly important role in anti-aging and diseases treatment fields (such as the treatments of diabetes, cancer, immune system disorders and ischemic tissue necrosis, etc.)

Following the rise of personalized cell therapy technology, now it is considered that fast proliferation of hematopoietic stem cells *in vitro* before reinfusion back into mammals, is beneficial to improve the functions of hematopoietic system in our body, including raising the numbers and functions of red blood cells and immune cells, maintaining blood sugar levels, improving anti-tumor capabilities and immune defense capabilities, and more, which mainly manifests in the improvements of disease treatment capabilities including anti-tumor capabilities, and it makes the organs and tissues younger, that prolongs our lives.

However, how to achieve a fast proliferation of hematopoietic stem cells while not affecting their functions becomes the key to hematopoietic stem cells culture, while in current arts, the hematopoietic stem cells culture technology is not mature enough, showing in the slow proliferation speed and low passage numbers.

Therefore, the prior art needs to be improved and developed.

The technical problem to be solved in the present invention, aiming at the defects of the prior art, provides a culture medium for hematopoietic stem cells and the applications thereof as well as the stem cells culture method, in order to solve the problems in the prior art, that the hematopoietic stem cells culturing *in vitro* are having a relatively slow proliferation speed and a low passage number.

The technical solution of the present invention to solve the said technical problems is as follows:
A hematopoietic stem cell culture medium, wherein, the said hematopoietic stem cell culture medium includes amino acids, vitamins, salts, lipids, protein polypeptides and cytokines;
   wherein, the said amino acids include one or several of alanine, arginine, asparagine, aspartic acid, cysteine, glutamic acid, glycine, histidine, isoleucine, leucine, lysine , methionine, phenylalanine, proline, serine, threonine, tryptophan, tyrosine, valine, glutamine and taurine;
The said vitamins include one or several of biotin, choline chloride, D-calcium pantothenate, Sodium D-pantothenate, folic acid, inositol, nicotinamide, pyridoxine hydrochloride, riboflavin, sulfur ammonia hydrochloride, coenzyme Q10, vitamin B12 , putrescine dihydrochloride, vitamin C and vitamin E;
The said salts include one or several of sodium bicarbonate, calcium chloride, potassium chloride, magnesium chloride, sodium chloride, sodium pyruvate, Edetate disodium (sodium salt of ethylenediaminetetraacetic acid), heparin sodium, β- mercaptoethanol and phenol red;
The said lipids include one or several of dexamethasone, oleic acid, cholesterol, ethanolamine, linoleic acid, lipoic acid and lipid mixtures.

The culture medium also includes protein polypeptides and/or cytokines; wherein, the said protein polypeptides and/or cytokines include one or several of fibroblast growth factor 1, fibroblast growth factor 2, epidermal growth factor, platelet-derived growth factor, insulin, insulin-like growth factor 1, vascular endothelial growth factor, placental growth factor, interleukin inhibitory factor, transferrin, human serum albumin, colony-stimulating factor 1, tumor necrosis factor, tumor transforming factor, macrophage colony-stimulating factor, granulocyte colony-stimulating factor, interleukin-2, interferon, erythropoietin, thrombopoietin, stem cell factor, insulin, reductive glutathione.

The said hematopoietic stem cell culture medium, wherein, the said hematopoietic stem cell culture medium includes the following components:

| | |
|---|---|
| Alanine | 0.05 mM |
| Arginine | 2.00 mM |
| Asparagine | 0.55 mM |
| Aspartic acid | 0.4 mM |
| Cysteine | 0.15 mM |
| Glutamate | 0.15 mM |
| Glycine | 0.25 mM |
| Histidine | 0.15 mM |
| Isoleucine | 2.50 mM |
| Leucine | 1.20 mM |
| Lysine | 1.00 mM |
| Methionine | 0.60 mM |
| Phenylalanine | 1.05 mM |
| Proline | 0.02 mM |
| Serine | 0.70 mM |
| Threonine, | 0.30 mM |
| Tryptophan | 0.10 mM |
| Tyrosine | 0.15 mM |
| Valine | 1.4 mM |
| Glutamine | 4 mM |
| Insulin | 2-10 mg / L |
| Transferrin, TRF | 5-50 mg/L |
| Human serum albumin | 0.1 g / L |
| Ascorbic acid | 50 mg / L |
| Biotin | 30µM |
| Choline chloride | 60µM |
| Folic acid | 6µM |
| Coenzyme Q10 | 1µM |
| Dexamethasone | 10 nM |
| D- Pan sodium | 5µM |
| Inositol | 70µM |
| Nicotinamide | 20µM |
| Pyridoxine hydrochloride | 0.15µM |
| Riboflavin | 0.55µM |
| Aneurine hydrochloride | 0.55µM |
| Vitamin B12 | 0.5µM |
| Putrescine dihydrochloride | 0.5µM |
| Vitamin C | 10 mg / L |
| Vitamin E | 0.25 mg / L |
| Heparin | 0.4g / L |
| D- glucose | 18 mM |
| Reduced glutathione | 0.001 mg / L |
| Taurine | 0.0072 g / L |
| β- mercaptoethanol, | 0.05 g / L |
| Phenol red | 8.1 mg / L |
| Oleic acid | 2.50 mg / L |
| Linoleic acid | 1.5 mg / L |
| Lipoic acid | 0.1 mg / L |
| Lipid mixture | 1 ml / L |
| Cholesterol | 5 mg / L |
| Ethanolamine | 60 µl / L |
| Sodium bicarbonate | 11.6 mM |
| Calcium chloride | 0.084 mM |
| Potassium chloride | 4.16 mM |
| Magnesium chloride | 0.3 mM |
| Magnesium sulfate | 0.407 mM |
| Sodium chloride | 120.61 mM |
| Sodium dihydrogen phosphate | 0.453 mM |
| Disodium hydrogen phosphate | 0.5 mM |
| Sodium pyruvate | 0.5 mM |
| Hematopoietic stem cell proliferation agent | 0.5 mg / L. |

The said hematopoietic stem cell culture medium, wherein, the said hematopoietic stem cell culture medium includes the following components:

| | |
|---|---|
| Epidermal growth factor | 1-10 µg / L |
| Fibroblast growth factor 1 | 1-10 µg / L |
| Fibroblast growth factor 2 | 1-10 µg / L |
| Insulin-like growth factor 1 | 1-10 µg / L |
| Leukemia inhibitory factor | 1-10 µg / L |
| Platelet-derived growth factor | 1-10 µg / L |
| Placental Growth Factor | 1-10 µg / L |
| TNF | 1-10 µg / L |
| Vascular endothelial growth factor | 1-10 µg / L |
| Colony-stimulating factor 1 | 1-10 µg / L |
| Macrophage colony stimulating factor | 1-10 µg / L |
| Granulocyte colony stimulating factor | 1-10 µg / L |
| Erythropoietin | 1-10 µg / L |
| Thrombopoietin | 1-10 µg / L |
| Stem cell factor | 1-10 µg / L |
| Interleukin -2 | 1-10 µg / L |
| Interferon | 1-10 µg / L. |

The said hematopoietic stem cell culture medium, wherein, the said hematopoietic stem cell culture medium also includes trace elements and/ or organic small molecules;
wherein, the said trace elements include one or several of cobalt chloride, sodium selenite, nickel chloride, manganese chloride, ammonium heptamolybdate, aluminum chloride, potassium chromium sulfate, copper sulfate, ferric nitrate, ferrous sulfate or zinc sulfate; and the said small organic molecules include one or several of β-mercaptoethanol, reduced glutathione, D-glucose, hematopoietic stem cell proliferation agent, taurine or sodium heparin.

The said hematopoietic stem cell culture medium, wherein, the said hematopoietic stem cell culture medium also includes plant extracts and/or animal tissue cell extracts after sterilized and pathogen-free processed for safety; wherein, the said plant extracts come from one or several of ginseng, astragalus, Chinese wolfberry, Chinese angelica and Codonopsis pilosula; and the said animal tissue cell extracts are one or several components extracted from blood plasma or serum.

The said hematopoietic stem cell culture medium, wherein, the said hematopoietic stem cell culture medium contains the following components:

| | |
|---|---|
| Ginseng extracts | 1-50mg/L |
| Astragalus extracts | 1-50mg/L |
| Chinese wolfberry extracts | 1-50mg/L |
| Chinese angelica extracts | 1-50mg/L |
| Plasma or serum from young animals | 1-5% v/v |

An implement of the hematopoietic stem cell culture medium, wherein, the said hematopoietic stem cell culture medium is configured to culture hematopoietic stem cells.

The said implement of the hematopoietic stem cell culture medium, wherein, the said hematopoietic stem cells are CD34+ cells isolated from the peripheral blood or umbilical cord blood.

A stem cell culture method using the hematopoietic stem cell culture medium, wherein, it includes the following steps:
A. Culture medium preparations: add amino acids, vitamins, salts, lipids, protein polypeptides and cytokines into the culture medium;
B. Hematopoietic stem cell preparations: remove the non-nuclear cells or multi-nuclear cells from the peripheral blood or umbilical cord blood and isolate CD34+ cells;
C, Cell culture: Inoculate the isolated CD34+ cells into the above said culture medium and start suspension culturing.

Benefits: the present invention describes a hematopoietic stem cell culture medium and the applications as well as the stem cell culture methods, which adopts amino acids, vitamins, salts, lipids, proteins peptides and cytokines as the basic medium, and adds one or several of the trace elements or organic small molecules, one or several of the plant extracts or animal tissue cell extracts, and together that composes the hematopoietic stem cell culture medium, which can quickly proliferate hematopoietic stem cells characterized in CD34+, making both the proliferation speed of the hematopoietic stem cells and the telomere length get improved a lot, comparing to cells collected from the conventional control culture medium; also, those stem cells cultured in the culture medium described in the present invention own the effects of lowering blood sugar, anti-tumors, extending the body life and else.

FIG. 1 illustrates the control diagram of the stem cells culture method based on the said hematopoietic stem cell culture medium described in the present invention.

FIG. 2-3 illustrates the result data comparison diagrams for embodiment 1 described in the present invention.

FIG. 4 illustrates the result data comparison diagrams for embodiment 2 described in the present invention.

FIG. 5 illustrates the result data comparison diagrams for embodiment 3 described in the present invention.

FIG. 6 illustrates the result data comparison diagrams for embodiment 4 described in the present invention.

FIG. 7 illustrates the result data comparison diagrams for embodiment 5 described in the present invention.

FIG. 8 illustrates the result data comparison diagrams for embodiment 6 described in the present invention.

The present invention provides a culture medium for hematopoietic stem cells and the applications thereof as well as the stem cells culture method, in order to make the purpose, technical solution and the advantages of the present invention clearer and more explicit, further detailed descriptions of the present invention are stated here, referencing to the attached drawings and some embodiments of the present invention. It should be understood that the detailed embodiments of the invention described here are used to explain the present invention only, instead of limiting the present invention.

The present invention provides a hematopoietic stem cell culture medium, which includes amino acids, vitamins, salts, lipids, protein polypeptides and cytokines. The components contained in the hematopoietic stem cell culture medium prepared following the method described in the present invention may provide enough nutrition for the hematopoietic stem cells culture, and accelerate the growth speed of the hematopoietic stem cell. Comparing to a conventional culture medium, the culture medium described in the present invention may not only proliferate stem cells with different origins in a faster speed and increase the passage number, but also keep the potential of stem cells as well.

Specifically, the said amino acids include one or several of alanine, arginine, asparagine, aspartic acid, cysteine, glutamic acid, glycine, histidine, isoleucine, leucine, lysine acid, methionine, phenylalanine, proline, serine, threonine, tryptophan, tyrosine, valine, glutamine and taurine. Amino acids are important materials required in cell lives, which may act as a nitrogen balance in cells, and may be converted into sugars or lipids, may participate in the constitution of enzymes, hormones and some vitamins, also, digestion and absorption of proteins are achieved with the help of amino acids. In a preferred embodiment, an immune cells culture medium described in the present invention has an addition of the above said 20 amino acids.

Additionally, vitamins, salts, lipids, protein polypeptides are all nutrition to life, and providing important actions to the growth of the hematopoietic stem cell. Specifically, the said amino acids include one or several of alanine, arginine, asparagine, aspartic acid, cysteine, glutamic acid, glycine, histidine, isoleucine, leucine, lysine acid, methionine, phenylalanine, proline, serine, threonine, tryptophan, tyrosine, valine, glutamine and taurine. The said vitamins include one or several of biotin, choline chloride, calcium D-pantothenate, Sodium D-pantothenate, folic acid, inositol, nicotinamide, pyridoxine hydrochloride, riboflavin, sulfur ammonia hydrochloride, coenzyme Q10, vitamin B12 , putrescine dihydrochloride, vitamin C and vitamin E. The said salts include one or several of sodium bicarbonate, calcium chloride, potassium chloride, magnesium chloride, sodium chloride, sodium pyruvate, Edetate disodium (sodium salt of ethylenediaminetetraacetic acid), heparin sodium, β- mercaptoethanol and phenol red. The said lipids include one or several of dexamethasone, oleic acid, cholesterol, ethanolamine, linoleic acid, lipoic acid and lipid mixtures.

Additionally, the hematopoietic stem cell culture medium as described in the present invention also includes protein polypeptides and/or cytokines; wherein, the said protein polypeptides and/or cytokines include one or several of fibroblast growth factor 1, fibroblast growth factor 2, epidermal growth factor, platelet-derived growth factor, insulin, insulin-like growth factor 1, vascular endothelial growth factor, placental growth factor, interleukin inhibitory factor, transferrin, human serum albumin, colony-stimulating factor 1, tumor necrosis factor, tumor transforming factor, macrophage colony stimulating factor, granulocyte colony-stimulating factor, interleukin-2, interferon, erythropoietin, thrombopoietin, stem cell factor, insulin and reductive glutathione.

In a preferred embodiment, the said hematopoietic stem cell culture medium also includes trace elements and/ or organic small molecules; wherein, the said trace elements include one or several of cobalt chloride, sodium selenite, nickel chloride, manganese chloride, ammonium heptamolybdate, aluminum chloride, potassium chromium sulfate, copper sulfate, ferric nitrate, ferrous sulfate or zinc sulfate; the said small organic molecules include β-mercaptoethanol, reduced glutathione, D-glucose, hematopoietic stem cell proliferation agent, taurine or sodium heparin.

Preferably, the hematopoietic stem cell culture medium described in the present invention also includes plant extracts and/ or animal tissue cell extracts after sterilized and pathogen-free processed for safety. Wherein, the said plant extracts come from one or several of ginseng, astragalus, Chinese wolfberry, Chinese angelica and Codonopsis pilosula; and the said animal tissue cell extracts come from the blood plasma or serum of animals having a relatively strong hematopoietic capability, such as the serum or plasma from a young deer.

The hematopoietic stem cell culture medium provided by the present invention is suitable for rapid culture of human or mammalian tissue-derived hematopoietic stem cells, the said hematopoietic stem cells are CD34+ cells isolated from the peripheral blood or umbilical cord blood. Specifically, the hematopoietic stem cells cultured in the hematopoietic stem cells culture medium described in the present invention are derived from the blood system of mammalian animals, include but not limited to human, mouse, rat, and these hematopoietic stem cells may be harvested by isolating from the blood or tissue organs in mammalian animals. The hematopoietic stem cells culture medium described in the present invention can improve the proliferation speed of hematopoietic stem cells 8 times above that of stem cells cultured in a conventional culture medium. The hematopoietic stem cells cultured in the hematopoietic stem cell culture medium described in the present invention own the same functions as those cultured in a conventional culture medium, that is, owning significant functions on lowering blood sugar, anti-tumor, and delaying aging process, also, the hematopoietic stem cell culture medium described in the present invention owns significant advantages in culture speeds, it may rapidly propagate the needed hematopoietic stem cells.

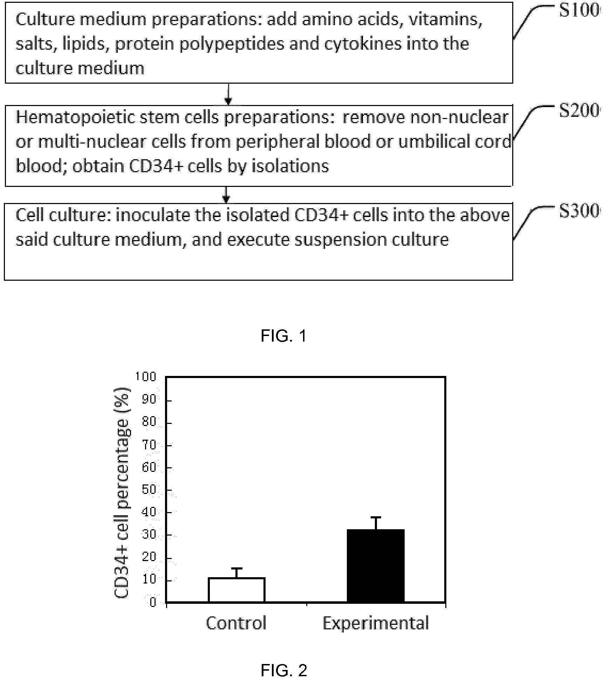

Additionally, the present invention also provides a stem cell culture method based on the above said hematopoietic stem cell culture medium, shown as Fig. 1, the specific steps are:
S100, culture medium preparations: add amino acids, vitamins, salts, lipids, protein polypeptides and cytokines into the culture medium in sequence. Of course, for this step S100, materials to add depend on the real conditions, for example, some extracts from plant cells or animal tissue cells may be added, and the detailed process is, extracting under high temperatures, and removing large insoluble particles by high-speed centrifugation, followed by filtering through 0.22µm and 0.1µm membrane filters, after the inactivation processes and removal of insoluble precipitates together with potential pathogens, the extracts are added to the said culture medium. Additionally, it may also add some trace elements or small organic molecules into the culture medium.
S200, hematopoietic stem cells preparations: Remove non-nuclear or multi-nuclear cells from peripheral blood or umbilical cord blood; obtain CD34+ cells by isolations. Specifically, add a certain amount of serum or plasma into the flask, remove non-nuclear or multi-nuclear cells by enriching mononuclear cells, and obtain mononuclear CD34+ cells
S300, cell culture: inoculate the isolated CD34+ cells into the above said culture medium, and execute suspension culture under suitable conditions.

Below are the further descriptions on the present invention through different embodiments, in the embodiments 1-5, the said hematopoietic stem cells culture medium, contains:

**Table 1: Components in the basic culture medium.**

| Ingredients | Concentration or amount |
|---|---|
| Ala (alanine) | 0.05 mM |
| Arg (Arg) | 2.00 mM |
| Asn (asparagine) | 0.55 mM |
| Asp (aspartic acid) | 0.4 mM |
| Cys (cysteine) | 0.15 mM |
| Glu (glutamic acid) | 0.15 mM |
| Gly (glycine) | 0.25 mM |
| His (histidine) | 0.15 mM |
| Ile (isoleucine) | 2.50 mM |
| Leu (leucine) | 1.20 mM |
| Lys (lysine) | 1.00 mM |
| Met (methionine) | 0.60 mM |
| Phe (phenylalanine) | 1.05 mM |
| Pro (Pro) | 0.02 mM |
| Ser (serine) | 0.70 mM |
| Thr (threonine) | 0.30 mM |
| Trp (tryptophan) | 0.10 mM |
| Tyr (tyrosine) | 0.15 mM |
| Val (valine) | 1.4 mM |
| Gln (glutamine) | 4 mM |
| Insulin | 2-10 mg/L |
| Transferrin | 5-50 mg/L |
| Human serum albumin (HSA) | 0.1 g/L |
| Ascorbic acid | 50 mg/L |
| Biotin (biotin) | 30 µM |
| Choline (Choline chloride) | 60 µM |
| Folate (Folic acid) | 6 µM |
| Coenzyme Q10 | 1 µM |
| Dexamethasone | 10 nM |
| D- Pan sodium (Na pantotenate) | 5 µM |
| Inositol (i-Inositol) | 70 µM |
| Nicotinamide | 20 µM |
| Pyridoxine hydrochloride | 0.15 µM |
| Riboflavin | 0.55 µM |
| Hydrochloric acid and sulfur ammonia (Thiamine hydrochloride) | 0.55 µM |
| Vitamin B12 | 0.5 µM |
| Putrescine dihydrochloride (Putresicine.2HCl) | 0.5 µM |
| Vitamin C | 10 mg/L |
| Vitamin E | 0.25 mg/L |
| Heparin (Heparin sodium) | 0.4 g/L |
| D- Glucose | 18 mM |
| Reduced Glutathione | 0.001 mg/L |
| Taurine | 0.0072 g/L |
| β- mercaptoethanol (BME) | 0.05 µg/L |
| Phenol red | 8.1 mg/L |
| Oleic acid (Oleic acid) | 2.50 mg/L |
| Linoleic acid (Linoleic acid) | 1.5 mg/L |
| Lipoic acid (Lipoic acid) | 0.1 mg/L |
| Lipid mixture (Lipid mixture) (Sigma, L5416) | 1 ml/L |
| Cholesterol (Cholesterol) | 5 mg/L |
| Ethanolamine (Ethanolamine) | 60 µl/L |
| Sodium bicarbonate (NaHCO3) | 11.6 mM |
| Calcium chloride (CaCl2) | 0.084 mM |
| Potassium chloride (KCl) | 4.16 mM |
| Magnesium chloride (MgCl2) | 0.3 mM |
| Magnesium sulfate (MgSO4) | 0.407 mM |
| Sodium chloride (Nacl) | 120.61 mM |
| Sodium dihydrogen phosphate (monohydrate) (NaH2PO4.H2O) | 0.453 mM |
| Disodium hydrogen phosphate (Na2HPO4) | 0.5 mM |
| Sodium pyruvate (Sodium pyruvate) | 0.5 mM |
| Hematopoietic stem cell proliferation | 0.5 mg/L |
| agent (StemRegenin-1 (RS1)) | |

**Table 2. Cytokines**

| | |
|---|---|
| Epidermal growth factor (EGF) | 1-10 µg/L |
| Fibroblast growth factor 1 (FGF1) | 1-10 µg/L |
| Fibroblast growth factor 2 (FGF2) | 1-10 µg/L |
| Insulin-like growth factor 1 (IGF1) | 1-10 µg/L |
| Leukemia inhibitory factor (LIF) | 1-10 µg/L |
| Platelet-derived growth factor (PDGF) | 1-10 µg/L |
| Placental Growth Factor (PGF) | 1-10 µg/L |
| Tumor necrosis factor (TNF) | 1-10 µg/L |
| Vascular endothelial growth factor (VEGF) | 1-10 µg/L |
| Colony-stimulating factor 1 (CSF1) | 1-10 µg/L |
| GM-CSF (macrophage colony stimulating factor) | 1-10 µg/L |
| G-CSF (granulocyte colony stimulating factor) | 1-10 µg/L |
| EPO (erythropoietin) | 1-10 µg/L |
| TPO (thrombopoietin) | 1-10 µg/L |
| SCF (stem cell factor) | 1-10 µg/L |
| IL2 (interleukin-2) | 1-10 µg/L |
| IFN (interferon) | 1-10 µg/L |

**Table 3. Concentrations of plant extracts and young animal blood extracts**

| | |
|---|---|
| Ginseng extracts | 1-50 mg/L |
| Astragalus extracts | 1-50 mg/L |
| Chinese wolfberry extracts | 1-50 mg/L |
| Chinese angelica extracts | 1-50 mg/L |
| Young animal blood plasma (or serum) | 1-5% v/v |

The embodiments described in the present invention are adopting the similar hematopoietic stem cell culture medium products (StemPro®-34 SFM) from Life Technologies, as the control of culture medium.

### Embodiment 1. Hematopoietic stem cell proliferation.

In order to check the effects of the hematopoietic stem cell culture medium described in the present invention to the hematopoietic stem cells proliferation speed, choose the peripheral blood 4 days after G-CSF mobilization, using Ficol method to prepare monocytes, and the monocytes obtained are cultured *in vitro,* either the culture medium in control (control group) or the hematopoietic stem cell culture medium (experiment group) is added respectively to either group after cells are inoculated at 10⁴ per hole, and both groups are cultured for 4-7 days (37°C, 5% CO₂) before flow sorting out the hematopoietic stem cells characterized with CD34+ using anti-CD34 antibodies, and the ratio of cells with CD34+ to total cells is calculated, while the total cells amount are detected with MTT method.

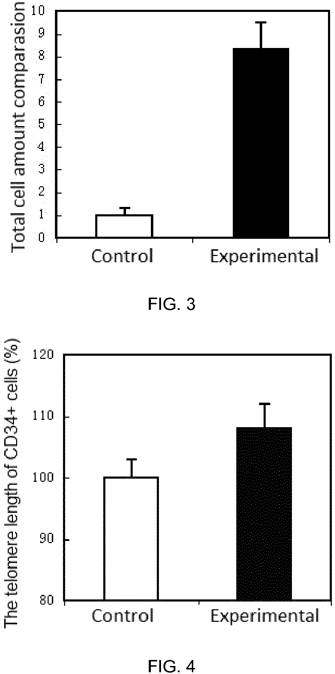

The results have shown that, under the same conditions, the ratio of the cells with CD34+ in the hematopoietic stem cell culture medium described in the present invention is above 30%, and 2.7 times to that in the control group (See Figure 2), while the amount of the total cells obtained in 1 liter of culture medium is 4.2 * 10⁹, and 8.3 times to that in the control group (See Fig. 3); the data is the average of 6 independent experiments ± SEM (n = 6; P <0.001).

### Embodiment 2. The improvement to the telomere length of CD34+ cells.

In order to check the effects of the hematopoietic stem cell culture medium described in the present invention to the telomere length of the hematopoietic stem cells, inject the mice with G-CSF and mobilize for 4 days, then collect the peripheral blood and prepare monocytes with Ficol method, and further using magnetic beads conjugated with anti-CD34 antibodies to enrich the CD34+ cells. Inoculate the CD34+ cells to the hematopoietic stem cell culture medium prescribed in the present invention (experiment group) and the conventional control culture medium (control group), based on 10⁴ per hole, then suspension culture for one week (37°C, 5% CO₂), before measuring the telomere length of the cultured cells using the real-time fluorescence quantitative PCR (RTFQ-PCR) method as described in the reference paper (Journal of Clinical and Experimental Medicine 2008 (7):14), the study results have shown that the telomere length of cells in experiment group is 8% longer than that in the control group. (See Fig. 4); the data are the average of 12 independent experiments ± SEM (n = 12; P <0.001).

### Embodiment 3: Functions of lowering blood sugar.

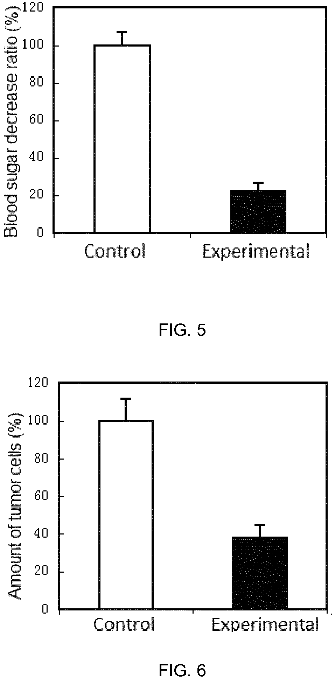

In order to check the treatment effects of the hematopoietic stem cells cultured in the hematopoietic stem cells culture medium described in the present invention to diabetes, select the hematopoietic stem cells collected from rats and execute cell culture experiments *in vitro,* cultured hematopoietic stem cells are intravenously injected into SD rats with STZ-induced diabetes (experiment group), while the rats in control group are injected with normal saline (NS) (control group), after one month, the blood sugar levels of STZ rats are examined. Results have shown that, the blood sugar level in the experiment group has reduced 78%, comparing to that in the control group. (See Fig. 5); the data are the average of 12 independent experiments ± SEM (n = 12; P <0.001).

### Embodiment 4: The killing effects to tumor cells.

Add the B-lineage acute lymphoblastic leukemia cell strain (Nalm-6) into RPMI-1640 complete culture medium composed by penicillin 100U/ml and streptomycin 100U/ml (15% by weight) inactivated newborn calf serum, and they are placed in an incubator at 37°C containing 5% CO₂ saturated humidity (volume percentage) to culture the cells. Adjust the concentration of cyclophosphamide into 10mg/ml with sterile PBS solution, and intraperitoneal inject the said cyclophosphamide into mice (2mg/mouse), continue for 2 days (2d); after 24 hours, collect the Nalm-6 cells locating in the logarithmic growth phase and centrifuge for 5 minutes at a speed of 1000r/min, then suspend in the sterile PBS solution, and adjust the Nalm-6 cell concentration to 2.5×10⁷ cells/ml, before intravenously injecting into mice tails (5×10⁶ cells each), and prepare the animal model of leukemia. For the hematopoietic stem cells experiment group, at the same time, inject the hematopoietic stem cells proliferated following the above mentioned method at an amount of 2.5×10⁵ cells per mouse, while no injections to the control group. Observe the symptoms every 2 or 3 days, and standardize the onset with hind limb paralysis, and record the mortality and morbidity date to mice. When the mice are dying, kill them by cervical dislocation and immediately collect various organs, then fix and dehydrate with neutral formalin (10% by weight), transparentizing, wax leaching, embedding, paraffin sectioning, and H-E staining before observing the tumor cell infiltration in each tissue under a light microscope. Bone marrow film preparation: remove the skin and muscle in the mice thighs and expose the femur as well as the joints connecting to both ends, then take the femur between both joints, cut one end of the femur, insert a 1ml syringe with NS deep into the bone marrow cavity and inject 0.5 ml NS, then drop the bone marrow flown out into the center of a clean glass slide, shake gently and spread it evenly, dry and fix it; stain the obtained bone marrow films with Wright's stain, and observe them under a light microscopy. Based on the statistics of the tumor cell amounts change before and after the hematopoietic stem cell treatment, it is founded that the tumor cell amounts after the injection of proliferated hematopoietic stem cells have reduced 62%, compared to that of non injections. (See Fig. 6); data are the average of 6 independent experiments ± SEM (n=6; P <0.001).

### Embodiment 6: Function on extending the older mice life

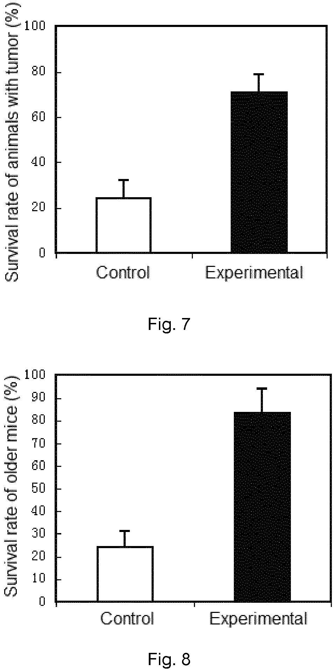

Buy 50 18-month-old female mice, divide into experimental and control groups equally. Each mouse in the experimental group subjects a tail vein injection of hematopoietic stem cells cultured in the hematopoietic stem cell culture medium described in the present invention, 5 x 10⁵ cells injected into each mouse tail vein and the same volume of NS is injected into the tail vein of each mouse in the control group, continue the same regular feeding and count the survival rate after one year. The statistical results have shown that, the survive rate has been increased to 83% in the experimental group, comparing to that in the control group of 24% (See Fig. 8). Data are the average of 25 independent experiments ± SEM (n=25; P <0.001).

In summary, embodiments described in the present invention adopt amino acids, vitamins, salts, lipids, protein polypeptides and cytokines, together with one or several of trace elements, small organic molecules, hematopoietic stem cell proliferation agents, plant extracts and animal tissue cell extracts, to compose a hematopoietic stem cell culture medium, which can quickly proliferate different species of CD34+ cells, and make both the proliferation speed and telomere length of CD34+ cells improve significantly; also, hematopoietic stem cells cultured in the hematopoietic stem cell culture medium described in the present invention may keep pretty good cell functions, which can achieve the functions include but not limited to anti-tumor, anti-aging, and extending lives, thus it has a pretty good application prospect.

It should be understood that, the implementations of the present invention are not limited to the above examples listed. It will be possible for a person skilled in the art to make modifications or replacements according to the above descriptions, which shall all fall within the protection scope of the appended claims of the present invention.

## Claims

1. A hematopoietic stem cell culture medium, wherein, the said hematopoietic stem cell culture medium includes amino acids, vitamins, salts and lipids; Wherein, the said amino acids include one or several of alanine, arginine, asparagine, aspartic acid, cysteine, glutamic acid, glycine, histidine, isoleucine, leucine, lysine, methionine, phenylalanine, proline, serine, threonine, tryptophan, tyrosine, valine, glutamine and taurine;
The said vitamins include one or several of biotin, choline chloride, D-calcium pantothenate, Sodium D-pantothenate, folic acid, inositol, nicotinamide, pyridoxine hydrochloride, riboflavin, sulfur ammonia hydrochloride, coenzyme Q10, vitamin B12 , putrescine dihydrochloride, vitamin C and vitamin E;
The said salts include one or several of sodium bicarbonate, calcium chloride, potassium chloride, magnesium chloride, sodium chloride, sodium pyruvate, Edetate disodium (sodium salt of ethylenediaminetetraacetic acid), heparin sodium, β- mercaptoethanol and phenol red;
The said lipids include one or several of dexamethasone, oleic acid, cholesterol, ethanolamine, linoleic acid, lipoic acid and lipid mixtures.
The culture medium also includes protein polypeptides and/or cytokines; wherein, the said protein polypeptides and/ or cytokines include one or several of fibroblast growth factor 1, fibroblast growth factor 2, epidermal growth factor, platelet-derived growth factor, insulin, insulin-like growth factor 1, vascular endothelial growth factor, placental growth factor, interleukin inhibitory factor, transferrin, human serum albumin, colony-stimulating factor 1, tumor necrosis factor, tumor transforming factor, macrophage colony stimulating factor, granulocyte colony-stimulating factor, interleukin-2, interferon, erythropoietin, thrombopoietin, stem cell factor, insulin, reductive glutathione.

2. The said hematopoietic stem cell culture medium according to claim 1, wherein, the said hematopoietic stem cell culture medium includes the following components:
| | |
|---|---|
| Alanine | 0.05 mM |
| Arg | 2.00 mM |
| Asparagine | 0.55 mM |
| Aspartic acid | 0.4 mM |
| Cysteine | 0.15 mM |
| Glutamate | 0.15 mM |
| Glycine | 0.25 mM |
| Histidine | 0.15 mM |
| Isoleucine | 2.50 mM |
| Leucine | 1.20 mM |
| Lysine | 1.00 mM |
| Methionine | 0.60 mM |
| Phenylalanine | 1.05 mM |
| Proline | 0.02 mM |
| Serine | 0.70 mM |
| Threonine, | 0.30 mM |
| Tryptophan | 0.10 mM |
| Tyrosine | 0.15 mM |
| Valine | 1.4 mM |
| Glutamine | 4 mM |
| Insulin | 2-10 mg / L |
| Transferrin, TRF | 5-50 mg / L |
| Human serum albumin | 0.1g / L |
| Ascorbic acid | 50 mg / L |
| Biotin | 30µM |
| Choline chloride | 60µM |
| Folic acid | 6µM |
| Coenzyme Q10 | 1µM |
| Dexamethasone | 10 nM |
| D- Pan sodium | 5µM |
| Inositol | 70µM |
| Nicotinamide | 20µM |
| Pyridoxine hydrochloride | 0.15µM |
| Riboflavin | 0.55µM |
| Aneurine hydrochloride | 0.55µM |
| Vitamin B12 | 0.5µM |
| Putrescine dihydrochloride | 0.5µM |
| Vitamin C | 10 mg / L |
| Vitamin E | 0.25 mg / L |
| Heparin | 0.4g / L |
| D- glucose | 18 mM |
| Reduced glutathione | 0.001 mg/L |
| Taurine | 0.0072 g / L |
| β- mercaptoethanol, | 0.05 g / L |
| Phenol red | 8.1 mg / L |
| Oleic acid | 2.50 mg / L |
| Linoleic acid | 1.5 mg / L |
| Lipoic acid | 0.1 mg/L |
| Lipid mixture | 1 ml / L |
| Cholesterol | 5 mg / L |
| Ethanolamine | 60 µl / L |
| Sodium bicarbonate | 11.6 mM |
| Calcium chloride | 0.084 mM |
| Potassium chloride | 4.16 mM |
| Magnesium chloride | 0.3 mM |
| Magnesium sulfate | 0.407 mM |
| Sodium chloride | 120.61 mM |
| Sodium dihydrogen phosphate | 0.453 mM |
| Disodium hydrogen phosphate | 0.5 mM |
| Sodium pyruvate | 0.5 mM |
| Hematopoietic stem cell proliferation agent | 0.5 mg / L. |

3. The said hematopoietic stem cell culture medium according to claim 1, wherein, the said hematopoietic stem cell culture medium includes the following components:
| | |
|---|---|
| Epidermal growth factor | 1-10 µg / L |
| Fibroblast growth factor 1 | 1-10 µg / L |
| Fibroblast growth factor 2 | 1-10 µg / L |
| Insulin-like growth factor 1 | 1-10 µg / L |
| Leukemia inhibitory factor | 1-10 µg / L |
| Platelet-derived growth factor | 1-10 µg / L |
| Placental Growth Factor | 1-10 µg / L |
| TNF | 1-10 µg / L |
| Vascular endothelial growth factor | 1-10 µg / L |
| Colony-stimulating factor 1 | 1-10 µg / L |
| Macrophage colony stimulating factor | 1-10 µg / L |
| Granulocyte colony stimulating factor | 1-10 µg / L |
| Erythropoietin | 1-10 µg / L |
| Thrombopoietin | 1-10 µg / L |
| Stem cell factor | 1-10 µg / L |
| Interleukin -2 | 1-10 µg / L |
| Interferon | 1-10 µg / L. |

4. The said hematopoietic stem cell culture medium according to claim 1, wherein, the said hematopoietic stem cell culture medium also includes trace elements and/ or organic small molecules;
wherein, the said trace elements include one or several of cobalt chloride, sodium selenite, nickel chloride, manganese chloride, ammonium heptamolybdate, aluminum chloride, potassium chromium sulfate, copper sulfate, ferric nitrate, ferrous sulfate or zinc sulfate;
the said small organic molecules include β-mercaptoethanol, reduced glutathione, D-glucose, hematopoietic stem cell proliferation agent, taurine or sodium heparin.

5. The said hematopoietic stem cell culture medium according to claim 1, wherein, the said hematopoietic stem cell culture medium also includes plant extracts and/ or animal tissue cell extracts after sterilized and pathogen-free processed for safety;
wherein, the said plant extracts come from one or several of ginseng, astragalus, Chinese wolfberry, Chinese angelica and Codonopsis pilosula; and the said animal tissue cell extracts come from one or several components in blood plasma or serum.

6. The said hematopoietic stem cell culture medium according to claim 5, wherein, the said hematopoietic stem cell culture medium contains the following components:
| | |
|---|---|
| Ginseng extracts | 1-50mg/L |
| Astragalus extracts | 1-50mg/L |
| Chinese wolfberry extracts | 1-50mg/L |
| Chinese angelica extracts | 1-50mg/L |
| Plasma or serum from young animals | 1-5% v/v |

7. An implement of the hematopoietic stem cell culture medium according to claim 1, wherein, the said hematopoietic stem cell culture medium is configured to culture hematopoietic stem cells.

8. The said implement of the hematopoietic stem cell culture medium according to claim 7, wherein, the said hematopoietic stem cells are CD34+ cells isolated from peripheral blood or umbilical cord blood.

9. A stem cell culture method with the hematopoietic stem cell culture medium according to claim 1, wherein, it includes the following steps:
A. Culture medium preparations: add amino acids, vitamins, salts, lipids, protein polypeptides and cytokines into the culture medium;
B. Hematopoietic stem cell preparations: remove the non-nuclear cells or multi-nuclear cells from the peripheral blood or umbilical cord blood and isolate CD34+ cells;
C, Cell culture: Inoculate the isolated CD34+ cells into the above culture medium for suspension cultures.
